# EUROPEAN PATENT SPECIFICATION

(11) **EP 4 048 159 B1**
(45) Date of publication and mention of the grant of the patent: **18.02.2026**
(21) Application number: 20878453.8
(22) Date of filing: 21.10.2020
(51) Int. Cl.: A61B 17/00, A61B 17/68, A61B 17/72, A61F 2/00, A61F 2/28, A61F 2/30, A61F 2/40

(54) **ORTHOPEDIC IMPLANT SYSTEM WITH AUGMENTATION DEVICE**
ORTHOPÄDISCHES IMPLANTATSYSTEM MIT AUGMENTATIONSVORRICHTUNG
SYSTÈME D'IMPLANT ORTHOPÉDIQUE DOTÉ D'UN DISPOSITIF D'AUGMENTATION

(30) Priority: 24.10.2019 US 201962925440 P
(43) Date of publication of application: 31.08.2022
(73) Proprietor: DePuy Ireland Unlimited Company, Ringaskiddy, County Cork (IE)
(72) Inventor: HODOREK, Brian C., Winona Lake, Indiana 46590 (US); PURDY, Matt J., Winona Lake, Indiana 46590 (US); PARROTT, Russ M., Winona Lake, Indiana 46590 (US); WIATER, J. Michael, Beverly Hills, Michigan 48025 (US); MURTHI, Anand M., Baltimore, Maryland 21218 (US); SMITH, Matthew J., Columbia, Missouri 65203 (US); CUFF, Derek J., Venice, Florida 34292 (US); JAWA, Andrew, Cambridge, Massachusetts 02139 (US); AUSTIN, Luke, Haddonfield, New Jersey 08033 (US)
(74) Representative: Murgitroyd & Company
(86) International application number: PCT/US2020/056575
(87) International publication number: WO 2021/081033

(56) References cited:
- WO-A1-2019/006205
- WO-A1-2019/053576
- WO-A1-2019/053576
- US-A1- 2005 065 526
- US-A1- 2008 183 297
- US-A1- 2012 221 111
- US-A1- 2013 123 929
- US-A1- 2015 305 877
- US-A1- 2016 045 323
- US-A1- 2016 045 323
- US-A1- 2016 317 307
- US-B2- 7 993 408

## Description

### FIELD OF THE INVENTION

The present invention relates generally to orthopedic surgery, implant systems used for replacing an articulation surface in a joint, such as, for example, shoulder prostheses. More specifically, but not exclusively, the present invention relates to glenoid implant systems for shoulder arthroplasties having augmentation devices to provide stability to a glenoid base plate when implanted over a glenoid deficiency in accordance with the appended claims.

### BACKGROUND OF THE INVENTION

Oftentimes during orthopedic surgery, a surgeon will encounter defects in a bone that the surgeon is operating on. For example, a surgeon will often encounter bone defects, such as voids or glenoid deficiencies, in various areas of a glenoid cavity during anatomic or reverse arthroplasty. Even after reaming preparation, defects may exist under the backsides of base plates of an orthopedic glenoid implant. Improper support may result in loosening and failure of the implant.

If a surgeon wanted complete backside support of the base plate, he could continue reaming deeper until one-hundred percent backside coverage is achieved (known as eccentric reaming). However, this often results in excessive removal of cortical bone (or cortical layer), i.e., the dense outer surface bone that forms a protective layer around the internal cavity of the bone. Studies have shown that cortical bone removal correlates with loosening of the implant over time.

Various augmented glenoid base plates exist that have predetermined shapes on their backsides. The predetermined shapes are designed to more closely fit various types of glenoid defects. However, these augmented base plates require extensive preparation of the glenoid cavity to create the angles and depths required to receive the augmented base plates. In addition, a large inventory of instruments and augmented implants with various backside shapes need to be brought into the operating room to cover whatever glenoid deficiency the surgeon may encounter. Moreover, the surgeon cannot determine which augmented base plate to use until the patient's glenoid is exposed.

Patient specific base plates, having a backside geometry that conforms to the patient's glenoid anatomy often require less preparation than preformed augmented base plates. However, to make such patient specific base plates, computer tomography (CT) scans are required on the patient to determine the shape of the customized base plate. This significantly increases the time and cost to the surgical procedure when compared to a conventional shoulder arthroplasty surgery. Additionally, custom devices are expensive and require an increased investment of time and resources on behalf of the company supplying the device.

Accordingly, there is a need for a base plate design and procedure that can provide modular stability to a base plate when bone defects are present without excessive bone removal. Additionally, there is a need for a base plate design and procedure that can provide such stability over a variety of defects with the same standard base plate. Moreover, there is a need to provide such stability without the need to manufacture a custom base plate that matches the patient's bone defects. Relevant teaching can be found in, for example, WO 2019/006205, US 2013/123929, WO 2019/053576, and US 2016/0045323.

### SUMMARY OF THE INVENTION

The invention is defined in claim 1.

Aspects of the present invention provides implant systems with augmentation devices for replacing an articulation surface in a joint, such as, for example, shoulder prostheses, in accordance with the appended claims. The augmentation device is used to provide stability to a base plate of an implant system when the base plate is located proximate to a defect, such as a void, in a bone. Also discussed herein for context are methods for using the implant systems.

In an aspect, provided herein is an implant system including a base plate having at least one thru-hole. A central screw is operable to extend from a lower surface of the base plate and anchor the base plate to a bone. At least one augmentation device is operable to extend through the at least one thru-hole. The augmentation device includes a post having a first end, a second end and a length sized to enable the augmentation device to traverse a defect in the bone when the base plate is anchored to the bone. A fastener portion is positioned on the first end of the post. The fastener portion is operable to fasten to the base plate. A footing portion is positioned on the second end of the post. The footing portion is operable to abut against and not penetrate a surface of the bone within the defect when the fastener portion is fastened to the base plate.

In another aspect, provided herein is an implant system including a base plate having a plurality of threaded peripheral holes disposed circumferentially around a central region of the base plate. A central screw is operable to extend from a lower surface of the base plate and threadingly anchor the base plate to a bone. At least one augmentation device is operable to extend through a first peripheral hole of the plurality of peripheral holes. The augmentation device includes a post having a first end, a second end and a length sized to enable the augmentation device to traverse a defect in the bone when the base plate is anchored to the bone. A threaded fastener portion of the augmentation device is positioned on the first end of the post. The fastener portion has threads configured to engage with threads of the first peripheral hole. A footing portion of the augmentation device is positioned on the second end of the post. The footing portion is operable to abut against and not penetrate a surface of the bone within the defect when the fastener portion is fastened to the base plate.

Also described herein is an augmentation device of an implant system, wherein the implant system has a base plate that is operable to be anchored to a bone. The augmentation device includes a post that is operable to extend through a thru-hole of the base plate. The post has a first end, a second end and a length sized to enable the augmentation device to traverse a defect in the bone when the base plate is anchored to the bone. A fastener portion is positioned on the first end of the post. The fastener portion is operable to fasten to the base plate. A footing portion is positioned on the second end of the post. The footing portion is operable to abut against and not penetrate a surface of the bone within the defect when the fastener portion is fastened to the base plate.

Also discussed herein and not claimed, is a method of surgically implanting an orthopedic implant into a bone. The method includes surgically exposing the surface of the bone. A circular surface is reamed into a portion of the bone. A center bore hole is drilled into the portion of the bone. The center bore hole and circular surface are concentric. A thru-hole of a base plate of an implant system is oriented over a defect in the bone. The base plate is inserted into the center bore hole such that the base plate is press-fit into the center bore hole and the thru-hole is located over the defect. An augmentation device is extended through the thru-hole. The augmentation device is fastened to the base plate such that a foot portion of the augmentation device abuts against, and does not penetrate, the surface of the bone within the defect.

These, and other objects, features and advantages of this invention will become apparent from the following detailed description of the various aspects of the invention taken in conjunction with the accompanying drawings.

### BRIEF DESCRIPTION OF THE DRAWINGS

The accompanying drawings, which are incorporated in and constitute a part of the specification, illustrate embodiments of the invention and together with the detailed description herein, serve to explain the principles of the invention. The drawings are only for purposes of illustrating preferred embodiments and are not to be construed as limiting the invention. It is emphasized that, in accordance with the standard practice in the industry, various features are not drawn to scale. In fact, the dimensions of the various features may be arbitrarily increased or reduced for clarity of discussion. The foregoing and other objects, features and advantages of the invention are apparent from the following detailed description taken in conjunction with the accompanying drawings in which:
FIG. 1 depicts a side perspective view of a defective glenoid cavity of a scapula bone;
FIG. 2 depicts a side perspective view of an orthopedic implant system, in accordance with an aspect of the present invention;
FIG. 3 depicts an exploded side view of the orthopedic implant system of FIG. 2, in accordance with an aspect of the present invention;
FIG. 4 depicts a top perspective view of a base plate of the orthopedic implant system of FIG. 2, in accordance with an aspect of the present invention;
FIG. 5 depicts a bottom perspective view of the base plate of FIG. 2, in accordance with an aspect of the present invention;
FIG. 6 depicts a side perspective view of a central screw of the orthopedic implant system of FIG. 2, in accordance with an aspect of the present invention;
FIG. 7 depicts a side perspective view of a coupling member of the orthopedic implant system of FIG. 2, in accordance with an aspect of the present invention;
FIG. 8 depicts a top perspective view of an embodiment of an augmentation device of the orthopedic implant system of FIG. 2 having a flat bottom footing portion, in accordance with an aspect of the present invention;
FIG. 9 depicts a side view of the augmentation device of FIG. 8, in accordance with an aspect of the present invention;
FIG. 10 depicts a side perspective view of another embodiment of an augmentation device having an arcuate bottom footing portion, in accordance with an aspect of the present invention;
FIG. 11 depicts a side perspective view of an example of an augmentation device having a conical bottom footing portion;
FIG. 12 depicts a side perspective view of another example of an augmentation device having a threaded bottom footing portion;
FIG. 13 depicts block diagram of a method of surgically preparing a bone to receive an orthopedic implant system according to the present invention;
FIG. 14 depicts a bottom perspective view of a bone reamer used to prepare a top surface of a glenoid cavity to accept an orthopedic implant system;
FIG. 15 depicts a top perspective view of a defective glenoid cavity, wherein the top surface of the glenoid cavity has been prepared to accept the orthopedic implant system of FIG. 2;
FIG. 16 depicts a side perspective view of the defective glenoid cavity of FIG. 15;
FIG. 17 depicts a side perspective view of a base plate of an orthopedic implant system being inserted into the prepared glenoid cavity of FIG. 15 with an insertion tool;
FIG. 18 depicts a side perspective view of the orthopedic implant system of FIG. 2 implanted into the glenoid cavity of FIG. 17;
FIG. 19 depicts a top perspective view of the orthopedic implant system of FIG. 2 having a glenosphere disposed over the base plate for use in a reverse shoulder arthroplasty, in accordance with an aspect of the present invention;
FIG. 20 depicts a bottom perspective view of the orthopedic implant system of FIG. 19, in accordance with an aspect of the present invention;
FIG. 21 depicts a side perspective view of the orthopedic implant system of FIG. 2, having a glenoid liner disposed over the base plate for use in an anatomic shoulder arthroplasty, in accordance with an aspect of the present invention;
FIG. 22 depicts a side perspective view on another example of an orthopedic implant system, in accordance with aspects of the present invention; and
FIG. 23 depicts a side exploded view of the orthopedic implant system of FIG. 22, in accordance with aspects of the present invention.

### DETAILED DESCRIPTION FOR CARRYING OUT THE INVENTION

Generally stated, disclosed herein are implant systems and methods of making the same. Further, surgical methods for using the implant systems are discussed.

In this detailed description and the following claims, the words proximal, distal, anterior, posterior, medial, lateral, superior and inferior are defined by their standard usage for indicating a particular part of a bone or implant according to the relative disposition of the natural bone or directional terms of reference. For example, "proximal" means the portion of a device or implant nearest the torso, while "distal" indicates the portion of the device or implant farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure.

As used herein, the word "exemplary" or "illustrative" means "serving as an example, instance, or illustration." Any implementation described herein as "exemplary" or "illustrative" is not necessarily to be construed as preferred or advantageous over other implementations. Moreover, in the present description, the terms "upper", "lower", "left", "rear", "right", "front", "vertical", "horizontal", and derivatives thereof shall relate to the invention as oriented in the first figure of each embodiment.

Similarly, positions or directions may be used herein with reference to anatomical structures or surfaces. For example, as the current implant systems (or implants), devices, systems and methods are described herein with reference to use with the bones of the shoulder, the bones of the shoulder and upper arm may be used to describe the surfaces, positions, directions or orientations of the implant systems, devices, systems and methods. Further, the implant systems, devices, systems and methods, and the aspects, components, features and the like thereof, disclosed herein are described with respect to one side of the body for brevity purposes. However, as the human body is relatively symmetrical or mirrored about a line of symmetry (midline), it is hereby expressly contemplated that the implant systems, devices, systems and methods, and the aspects, components, features and the like thereof, described and/or illustrated herein may be changed, varied, modified, reconfigured or otherwise altered for use or association with another side of the body for a same or similar purpose without departing from the scope of the claims. For example, the implant systems, devices, systems and methods, and the aspects, components, features and the like thereof, described herein with respect to the right shoulder may be mirrored so that they likewise function with the left shoulder and vice versa. Further, the implant systems, devices, systems and methods, and the aspects, components, features and the like thereof, disclosed herein are described with respect to the shoulder for brevity purposes, but it should be understood that the implant systems, devices, systems and methods may be used with other bones of the body having similar structures, for example the lower extremity, and more specifically, with the bones of the ankle, foot, and leg.

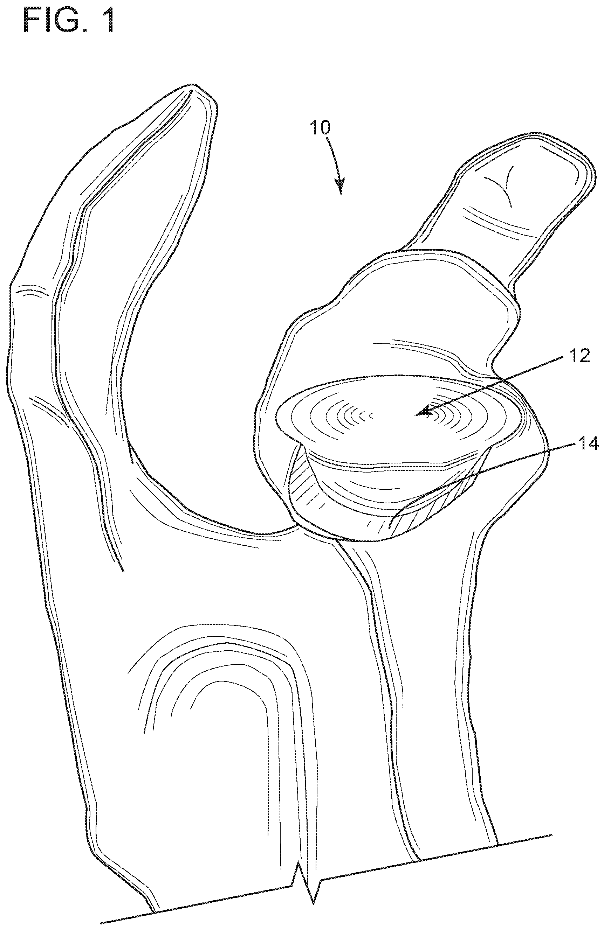

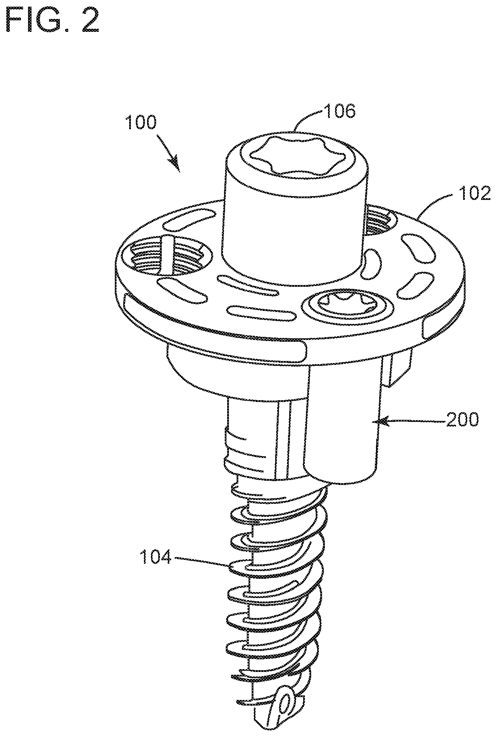

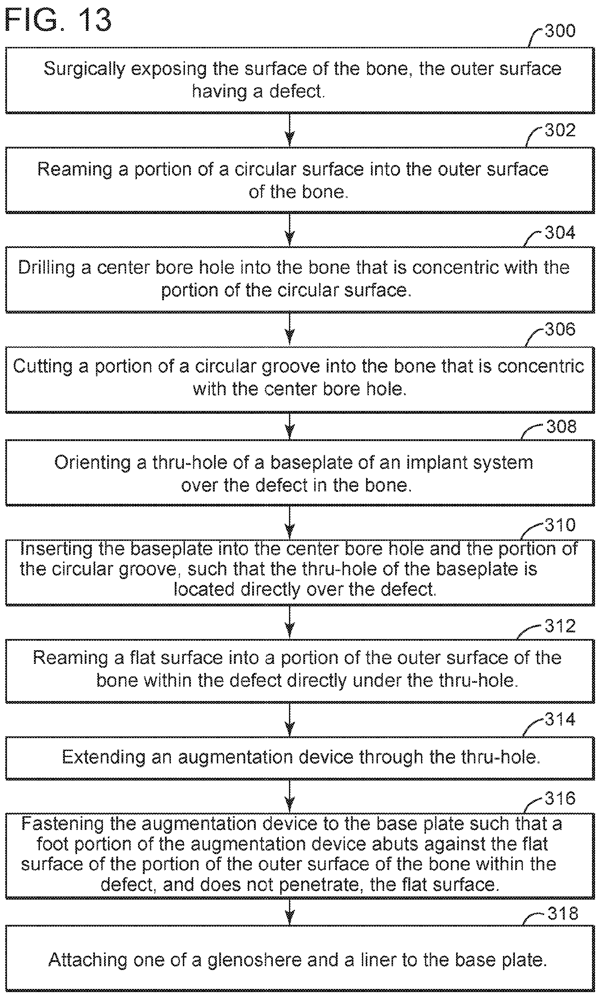

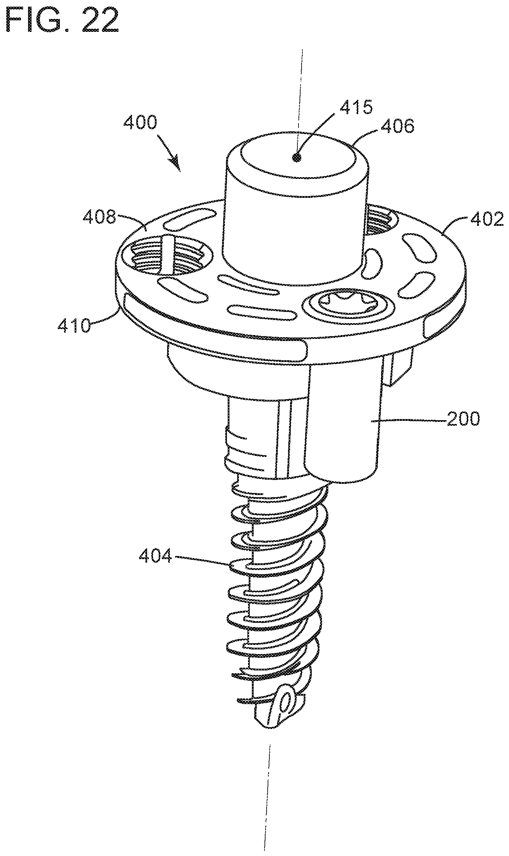

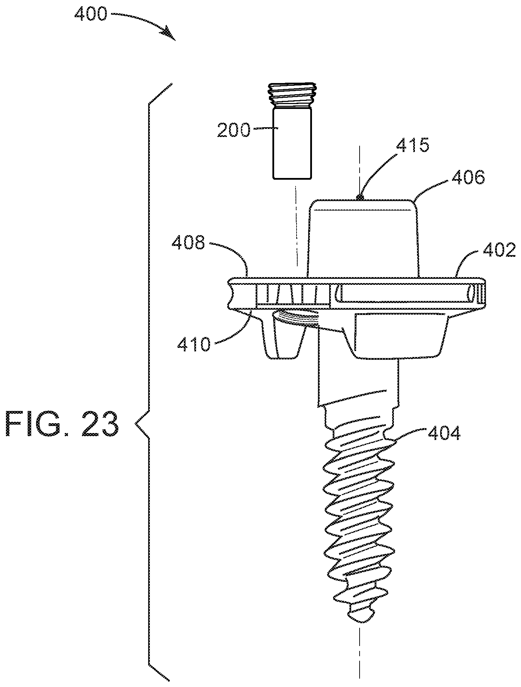

Referring to the drawings, like reference numerals are used to indicate like or analogous components throughout the several views. Particularly, FIG. 1 illustrates one example of a defective glenoid cavity. FIGS. 2-10 illustrate various views of an implant system in accordance with aspects of the present invention. Additionally, FIGS. 13-18 illustrate various steps and devices utilized in a method of preparing a bone for an orthopedic implant system according to the present invention. FIGS. 22 and 23 illustrate various views of an example of another embodiment of an implant system, wherein a base plate and central screw of the implant system are formed as a single monoblock construct.

Referring to FIG. 1, an example of a lateral perspective view of a defective glenoid cavity 12 of a scapula 10 is depicted. The glenoid cavity 12 includes at least one bone defect 14 that has been developed over time in a patient. For example, the bone defect 14 may be, without limitation, a void. The defect consists of a depression beyond the normal anatomy of the glenoid cavity.

Though the example of a defective scapula 10 illustrated in FIG. 1 shows one defect 14, two or more defects are also possible. Such defects may vary in depth and size depending on the conditions that caused them. Additionally, though the figures herein illustrate a glenoid cavity 12 with a defect, other bones in the human anatomy may also have similar defects that may be treatable within the scope of the present invention.

Such defects provide irregular surfaces in the glenoid cavity 12, thereby reducing structural support for the backside of base plates of orthopedic implant systems (or implants), even after reaming the glenoid cavity's articulating surface. If a surgeon wanted complete backside support of the base plate, the surgeon would have to continue reaming deeper until one-hundred percent backside coverage is achieved (known as eccentric reaming). However, this often results in excessive removal of the cortical layer. Studies have shown that cortical layer removal correlates with loosening of the implant over time. The cortical layer (or cortical bone) herein is the dense outer surface of the bone that forms a protective layer around the internal cavity of the bone.

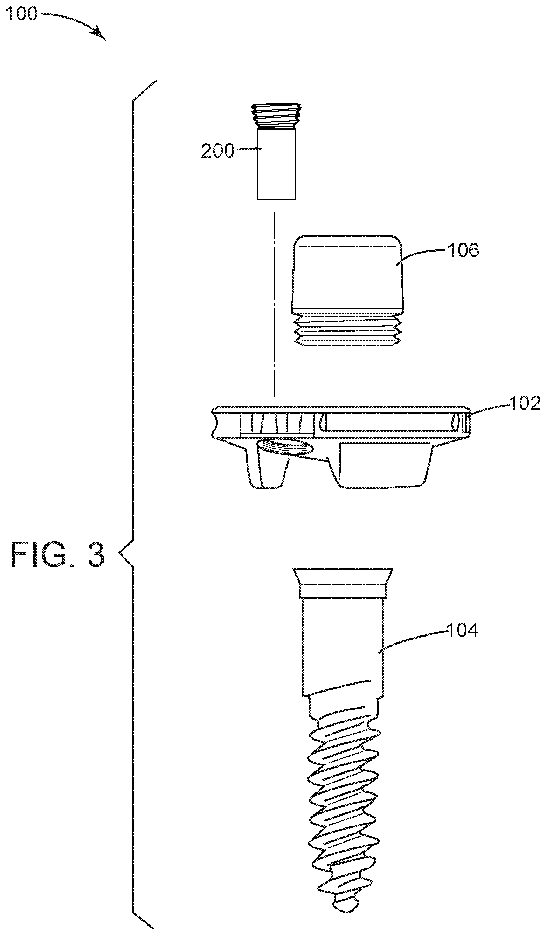

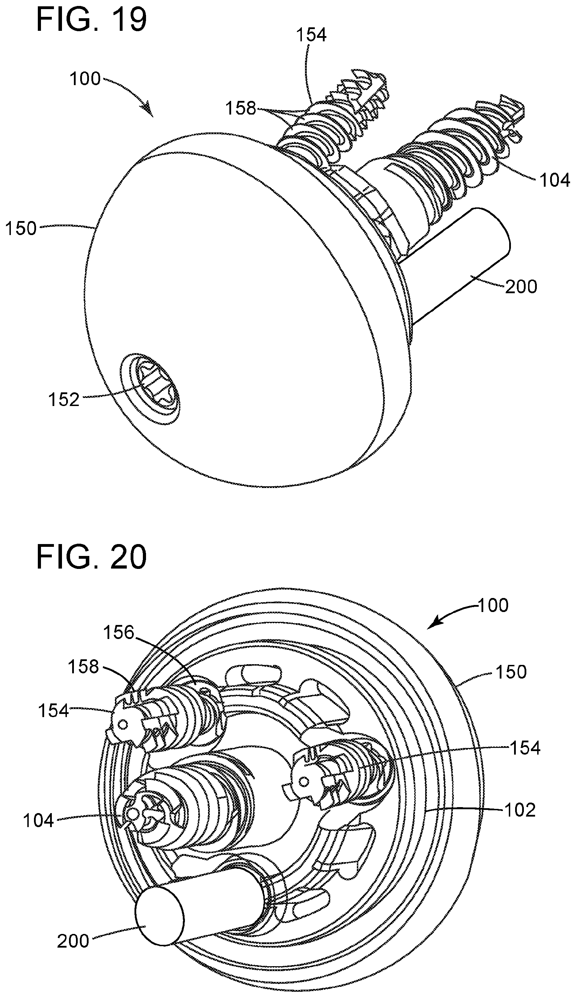

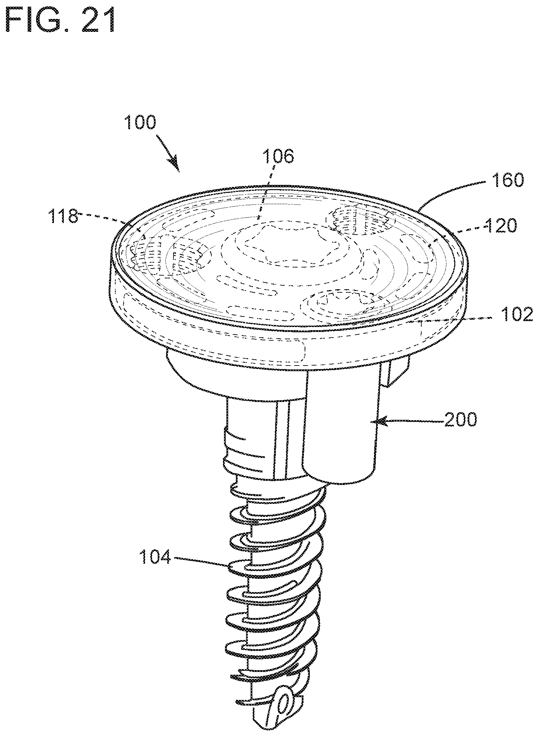

Referring to FIGS. 2 and 3, a side perspective view (FIG. 2) and an exploded view (FIG. 3) of an orthopedic implant system 100 is depicted, in accordance with an aspect of the present invention. Implant system 100 includes a base plate 102, a central screw 104, a coupling member 106, and at least one augmentation device 200. The implant system 100 may also include one or more peripheral screws 154 (seen in FIGS. 19 and 20). Further, the implant system may include a glenoid sphere 150 (seen in FIGS. 19 and 20) for use in a reverse shoulder arthroplasty and/or a glenoid liner 160 (seen in FIG. 21) for use in an anatomic shoulder arthroplasty.

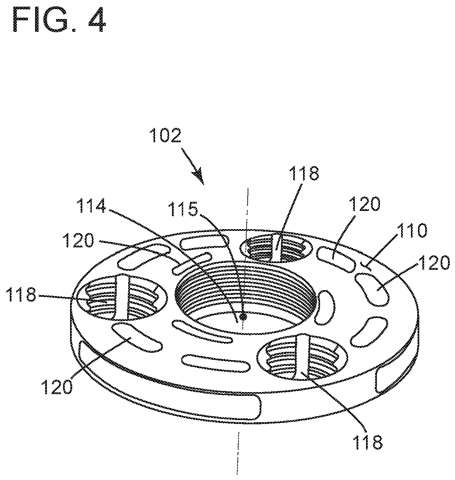

In the example of the implant system 100 illustrated in FIGS. 2 and 3, the base plate 102, central screw 104 and coupling member 106 are separate pieces. Additionally, the central screw 104 is separable from the base plate 102 and operable to extend through a central bore 114 (see FIG. 4), which is positioned in a central region 115 of the base plate 102. However, another example of an implant system 400 is illustrated in FIGS. 22 and 23 that is also within the scope of the present invention. In implant system 400, the base plate 402, central screw 404 and coupling member 406, may be integrally connected as a single monobloc construct.

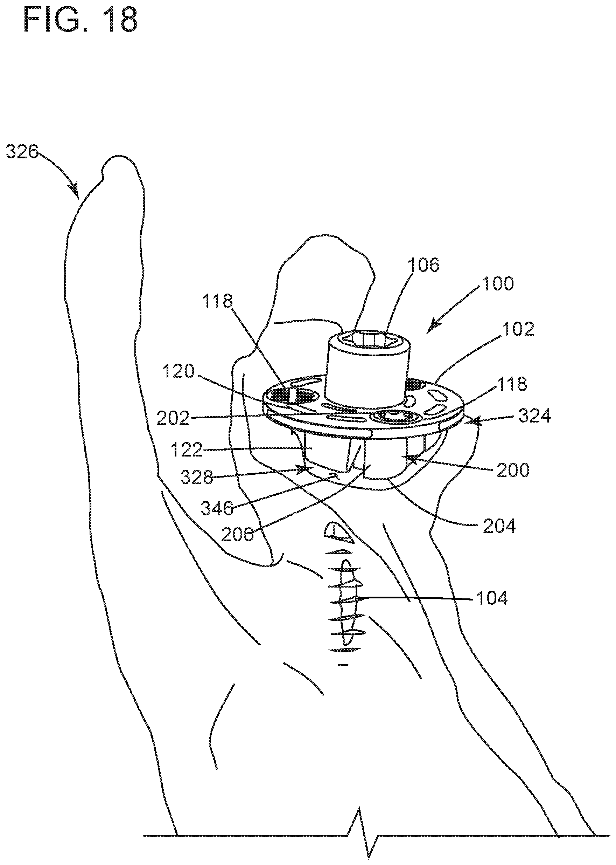

As will be explained in greater detail herein, the augmentation device 200 provides stability to the implant system 100, when the implant system is inserted into a glenoid cavity 324 having a bone defect 328, such as a void (seen in FIG. 18). The augmentation device 200 can be fastened to the base plate 102 and extend into the bone defect 14 such that a footing end of the augmentation device abuts against the surface of the bone within the defect 14. The bone within the defect 14 may include a hard bone, for example, cortical bone. The augmentation 200 can provide support to the base plate 102, much like the legs of a table provide support for the table, with minimal bone removal from the surface of the bone within the defect 14.

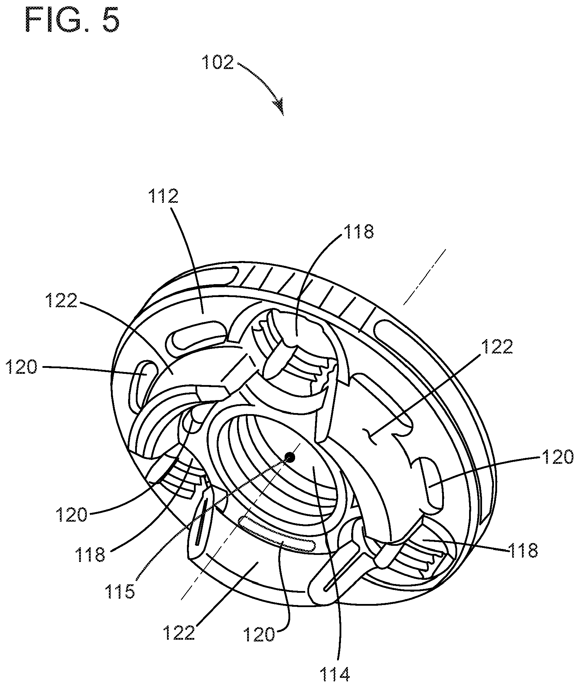

Referring to FIGS. 4 and 5, a top perspective view (FIG. 4) and a bottom perspective view (FIG. 5) of the base plate 102 of implant system 100 is depicted, in accordance with an aspect of the present invention. The base plate 102 may have, for example, a cylindrical shape with a circular top surface 110 and a circular bottom surface 112.

The base plate 102 may include a threaded central bore 114 disposed or positioned in a central region 115 of the base plate 102. The base plate 102 also includes at least one thru-hole. In this particular example, the base plate 102 includes two types of thru-holes, i.e., a plurality of threaded peripheral holes 118 and a plurality of arcuate slots 120. Both the plurality of peripheral holes 118 and the plurality of arcuate slots 120 are disposed circumferentially around the central bore 114 and/or central region 115 of the base plate.

Though only two types of thru-holes are illustrated in the examples herein (i.e., peripheral holes 118 and arcuate slots 120), other types of thru-holes are within the scope of the present invention. For example, the thru-holes may be one or more unthreaded holes or straight slots drilled through the base plate 102.

The plurality of threaded peripheral holes 118 are configured to engage with peripheral screws 154 (see FIGS. 19 and 20). During operation, at least one peripheral screw154 extends through any one of the plurality of peripheral holes 118 to further anchor the base plate 102 to a bone. As shown in FIG. 20, the peripheral screws 154 include first threads 156 and second threads 158. The first threads 156 are configured to engage with threads of any peripheral hole 118 of the plurality of peripheral holes 118. The second threads 158 are configured to screw into the bone and help anchor the base plate 102 to the bone.

FIG. 5 shows the base plate 102 having a plurality of arcuate protrusions 122 extending from the bottom surface 112 of the base plate 102 that are also disposed circumferentially around the central bore 114. The protrusions 122 may be comprised of a porous metal structure to provide sites for bone in growth when inserted into a prepared glenoid cavity.

As seen in FIG. 5, the plurality of arcuate slots 120 may be positioned adjacent to the inner and outer side walls of the protrusions 122 and my follow the profile of the protrusions 122. The arcuate slots 120, may potentially be utilized for revising the implant system 100, if required. For example, the arcuate slots 120 may allow a surgical instrument, such as an osteotome, to be passed down through the slots 120 to disengage away the metal to bone bond to allow the implant system 100 to be removed during a revision operation.

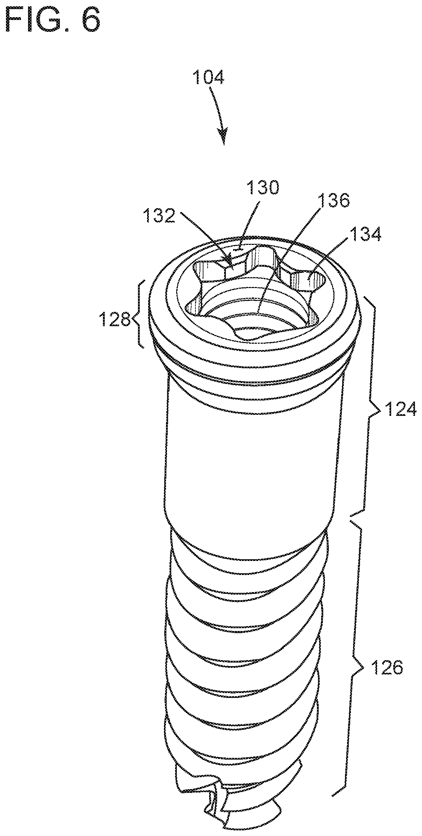

Referring to FIG. 6, a side perspective view of the central screw 104 of the orthopedic implant system 100 is depicted, in accordance with an aspect of the present invention. The central screw 104 is operable to extend through the central bore 114 and threadingly anchor the base plate 102 to a bone (see FIG. 18). In this example, the bone that the central screw 104 engages with is the glenoid cavity of a scapula (see FIG. 18).

As shown in FIG. 6, the central screw 104 may include a proximal non-threaded section 124 and a distal threaded section 126. The proximal section 124 may include a flared head portion 128 positioned at a first end 130 of the central screw 104. The proximal section 124 may also include a recess 132 extending into the proximal section 124 from the first end 130 of the central screw 104.

FIG. 6 shows that the recess 132 may include a first socket portion 134 and a second female threaded portion 136. The first socket portion 134 may extend from the first end 130 of the central screw 104 toward the second female threaded portion 136. The second female threaded portion 136 may extend from a bottom end of the first socket portion 134 toward the distal threaded section 126. The first socket portion 134 may be, for example, a driver feature for engaging a tool for inserting or removing the central screw 104. The second female threaded portion 136 may be, for example, threaded for receiving corresponding threads of a post 152 (see FIG. 19). The proximal section 124 may have, for example, a texture or coating to provide for porous fixation. The proximal section 124 may be, for example, configured or sized and shaped to conserve bone. The distal threaded section 126 may be, for example, threaded to engage a patient's bone to secure the base plate 102 to the patient's bone, such as, the glenoid cavity 12.

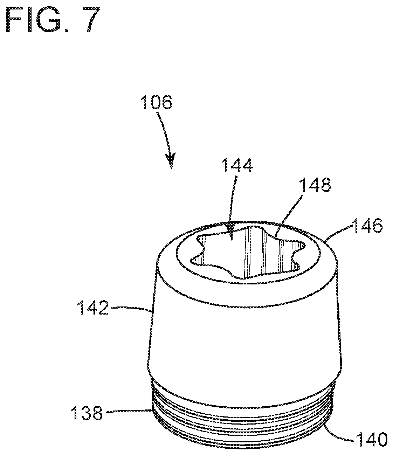

Referring to FIG. 7, a side perspective view of coupling member 106 of the orthopedic implant system 100 is depicted, in accordance with an aspect of the present invention. The coupling member 106 engages the base plate 102 via a threaded portion 138 to secure the central screw 104 within the central bore 114 of the base plate 102.

FIG. 7 shows the coupling member 106 including a distal male threaded section 138 terminating at a bottom end 140 of the coupling member 106. The coupling member 106 may also include a proximal unthreaded section 142 having a central thru-hole 144 extending through the coupling member 106 from a top end 146 to the bottom end 140. The central thru-hole 144 may include a socket portion 148 extending through at least a portion of the central thru-hole 144 from the top end 148. The socket portion 148 may be, for example, a drive feature for engaging a tool for inserting or removing the coupling member 106 into the central bore 114 of the base plate 102. The male threaded section 138 may be, for example, threaded for receiving corresponding female threads within the central bore 114. When threaded into the central bore 114, the bottom end 140 of coupling member 106 may abut against the flared head portion 128 of the central screw 104 to secure and lock the central screw 104 into place.

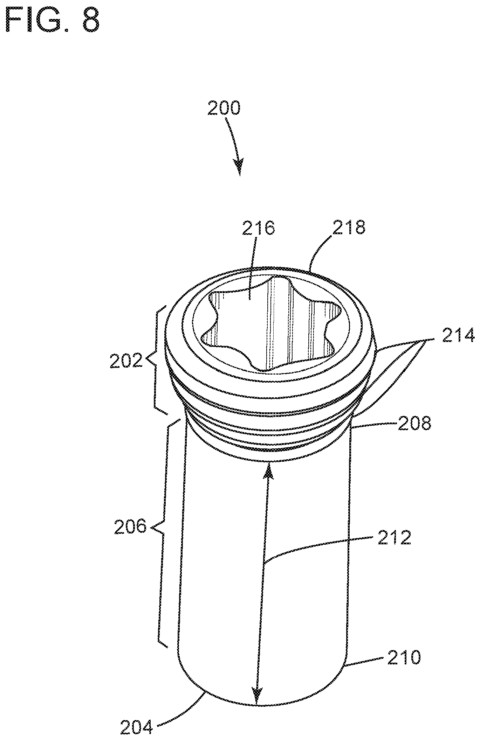

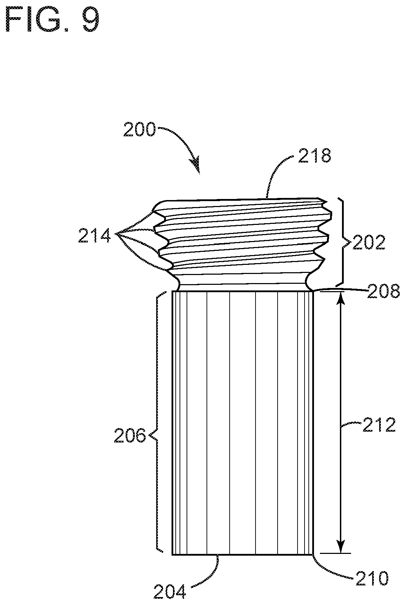

Referring to FIGS. 8 and 9, a top perspective view (FIG. 8) and a side view (FIG. 9) of a first example of an augmentation device 200 of the orthopedic implant system 100 is depicted in accordance with an aspect of the present invention. The augmentation device 200 includes a fastener portion 202 and a footing portion 204 connected therebetween by a post 206.

The augmentation device 200 is operable to extend through at least one thru-hole 118 of the base plate 102. In the examples illustrated herein, the augmentation device 200 is configured to extend through any one of the peripheral holes 118. However, the augmentation device 200 may be configured to extend through any one of the arcuate slots 120. Moreover, the augmentation device 200 may be configured to extend through any other type of thru-hole disposed within the base plate 102.

As shown in FIG. 8, the post 206 includes a first end 208, a second end 210 and a length 212 sized to enable the augmentation device 200 to traverse or fill a defect in a bone. For example, the length 212 of post 206 may be long enough such that when the augmentation device 200 is fastened to base plate 102 and anchored into the scapula 10, the augmentation device 200 may extend into the defect 14 of glenoid cavity 12 to abut its footing portion 204 against a bottom surface of the bone within the defect 14 (see FIG. 18) and provide support to the base plate 102. The post 206 may include a porous surface to provide sites for bone ingrowth when inserted into a prepared glenoid cavity. The porous surface may be comprised of, for example, a porous metal structure, or a porous coating. Though the post 206 is illustrated in FIGS. 8 and 9 as being cylindrical in shape, the post 206 may be configured in other shapes as well. For example, and without limitation, the post 206 may have a square or rectangular cross-sectional shape. Alternatively, the post 206 may have a shape that conforms to the shape of the thru-hole it is extended though.

FIG. 8 also shows the fastener portion 202 as being positioned on the first end 208 of the post 206. The fastener portion 202 couples the augmentation device 200 to the base plate 102. For example, the fastener portion 202 may include male threads 214 that are configured to engage with the female threads on any one of the peripheral holes 118 of the base plate 102. However, the fastener portion may also be configured to engage with any one of the arcuate slots 120 of the plurality of arcuate slots 120. For example, a first arcuate slot 120 may have portions of female threads 214 disposed on opposing side walls of the first arcuate slot 120 for engagement with male threads disposed on the fastener portion 202. Moreover, the fastener portion 202 may be configured to engage with any other thru-hole found in the base plate 102. Additionally, the fastener portion 202 may not include threads, and may be configured to be press-fit or cemented into a thru-hole.

The fastener portion 202 may include various components that are resilient, or spring loaded, and are capable of snapping into place and securing the augmentation device 200 when it is in a predetermined position. For example, the fastener portion 202 may include a top rim disposed at the top end 218 of the fastener portion 202 and a well-known spring loaded retractable retaining ring (or snap ring) disposed in a groove on the lower end of fastener portion 202 adjacent the top first end 208 of the post 206. The retaining ring (not shown) may retract radially into the groove when it is being inserted through a top of a thru-hole, and radially snap back partially out of the groove when it exits the bottom of the thru-hole. The retaining ring and top rim may be spaced apart to capture the base plate between them and to hold the augmentation device 200 in place once the augmentation device 200 extends through the thru-hole.

A socket portion 216 may be recessed into a top end 218 of the fastener portion 202. The socket portion 216 may be, for example, a drive feature for engaging a tool for inserting or removing the augmentation device 200. In this particular example, a tool may engage the socket portion 216 to thread the male threads 214 of the fastener portion 202 into the female threads on any one of the peripheral holes 118 of the base plate 102.

The footing portion 204 is positioned on the second end 210 of the post 206. The footing portion 204 is operable to abut against a surface of the bone within the defect 14 when the fastener portion 202 is fastened to the base plate 102. In the example illustrated in FIGS. 8 and 9, the footing portion 204 comprises a flat bottom footing portion. That is, the footing portion 204 is the flat surface at the bottom second end 210 of the post 206. The flat bottom footing portion 204 is operable to pressingly engage and not penetrate the surface of the bone within the defect 14.

As will be described in greater detail herein, a flat surface 344 may be reamed into a portion of the surface of the bone within the defect 14, directly under the thru-hole that the augmentation device 200 is inserted into. The flat surface 344 may rest against the flat bottom footing portion 204 of augmentation device 200 to provide support for the footing portion 204 along substantially the entire surface area of the flat bottom footing portion 204 and base plate 102.

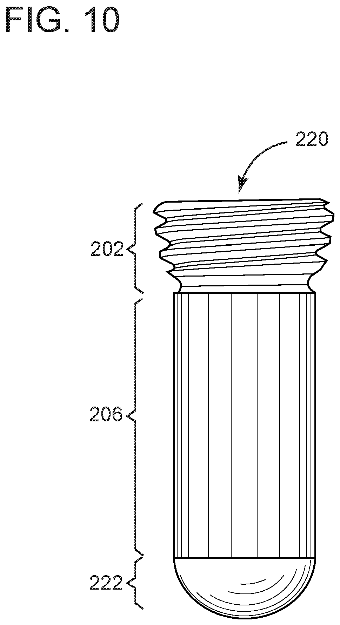

FIG. 10 shows a side perspective view of another example of an augmentation device 220 having an arcuate bottom footing portion 222, in accordance with an aspect of the present invention. The fastener portion 202 and the post 206 of the augmentation device 220 are as described above with reference to the augmentation device 200 and will not be described again here for brevity's sake.

The footing portion 222 has an arcuate or curved shape. The footing portion 222 is configured to press against and not penetrate the surface of the bone within the defect 14. The arcuate bottom footing portion 222 may abut against the bone within the defect 14 over a smaller percentage of its surface area than that of the flat bottom footing portion 204. As such, there may be less reaming required and decreased bone removal, relative to the flat bottom footing portion 204, to prepare the surface of the bone within the defect 14 to receive and support the arcuate bottom footing portion 222.

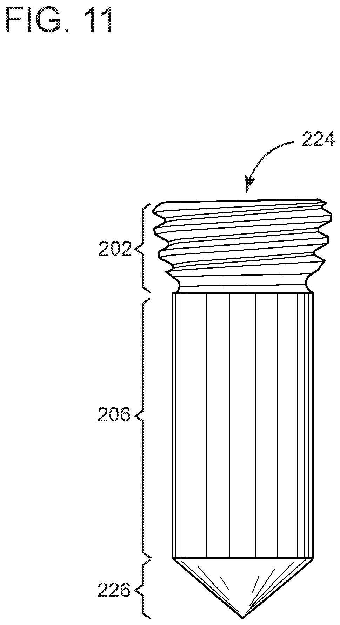

FIG. 11 is a side perspective view of another example of an augmentation device 224 having a conical bottom footing portion 226. The fastener portion 202 and the post 206 of the augmentation device 224 are as described above with reference to the augmentation device 200 and will not be described again here for brevity's sake. The footing portion 226 has a conical shape and again presses against the surface of the bone within the defect 14. The conical bottom footing portion 226 may abut against the bone within the defect 14 over an even smaller percentage of its surface area than that of the flat bottom footing portion 204. As such, there may be less reaming and bone removal required, relative to the flat bottom footing portion 204, to prepare the surface of the bone within the defect 14.

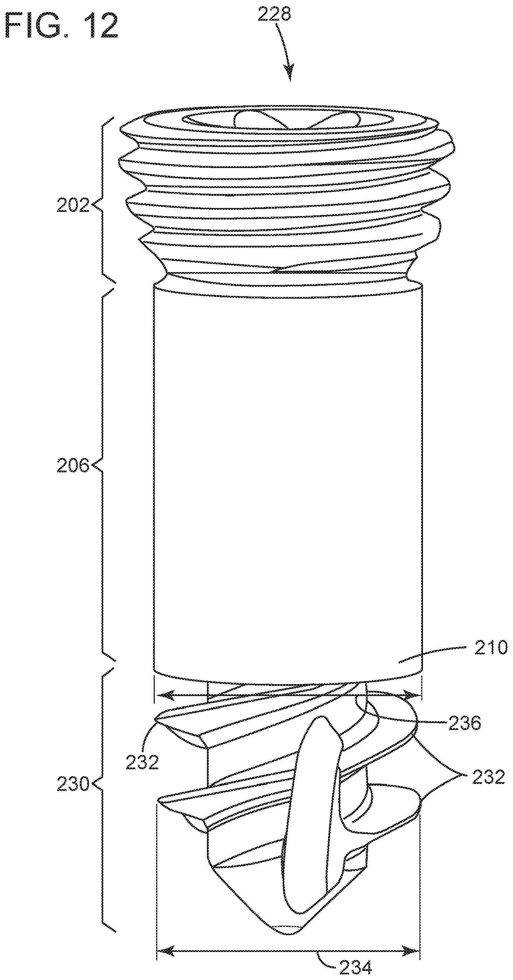

FIG. 12 shows a side perspective view of another example of an augmentation device 228 having a threaded bottom footing portion 230. The fastener portion 202 and the post 206 of the augmentation device 228 are as described above with reference to the augmentation device 200 and will not be described again here for brevity's sake. The threaded bottom footing portion 230 of the augmentation device 228 includes threads 232 that are designed to minimally penetrate the bone within the defect 14. For example, the augmentation device 228 may include at least one thread 232 and have a major diameter 234 that is less than or equal to the outer diameter 236 of the second end 210 of the post 206. Additionally, the threaded bottom footing portion 230 may include a limited number of threads, for example, no more than three threads 232, to limit the penetration into the surface of the bone within the defect 14. The threaded bottom footing portion 230 is operable to be threaded into the surface of the bone within the defect 14.

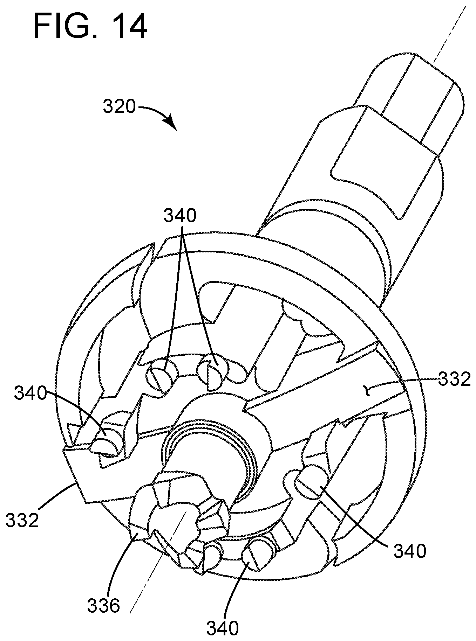

Referring to FIGS. 13-18, various steps utilized in a method of preparing a bone for the orthopedic implant system 100 are depicted. In this particular case, the method will be used to implant the orthopedic implant system 100 in the glenoid cavity 12 of a scapula 10. However, the method may be used to implant other orthopedic implant systems within the scope of this invention into other bones as well. More specifically, FIG. 13 depicts a block diagram of a method of surgically preparing a bone to receive the orthopedic implant system in accordance with the present invention and FIGS 14-21 support the various steps of the method.

Referring to FIG. 14, a bottom perspective view of a bone reamer 320 used to prepare a top (or outer) surface 322 of a glenoid cavity 324 having a bone defect 328, such as a void, to accept the orthopedic implant system 100 is depicted. Similar such bone reamers are disclosed in: international application no. PCT/US2019/051322, filed on September 16, 2019.

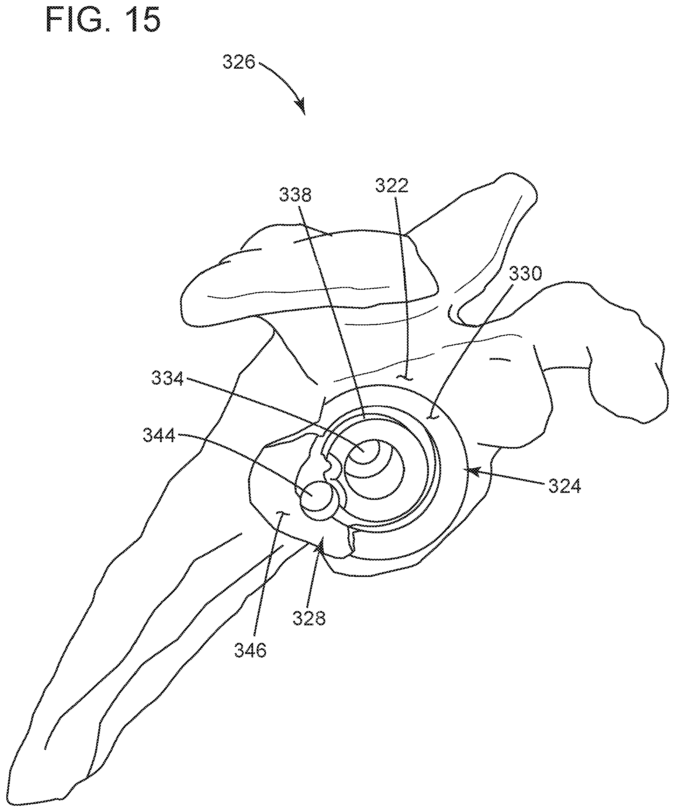

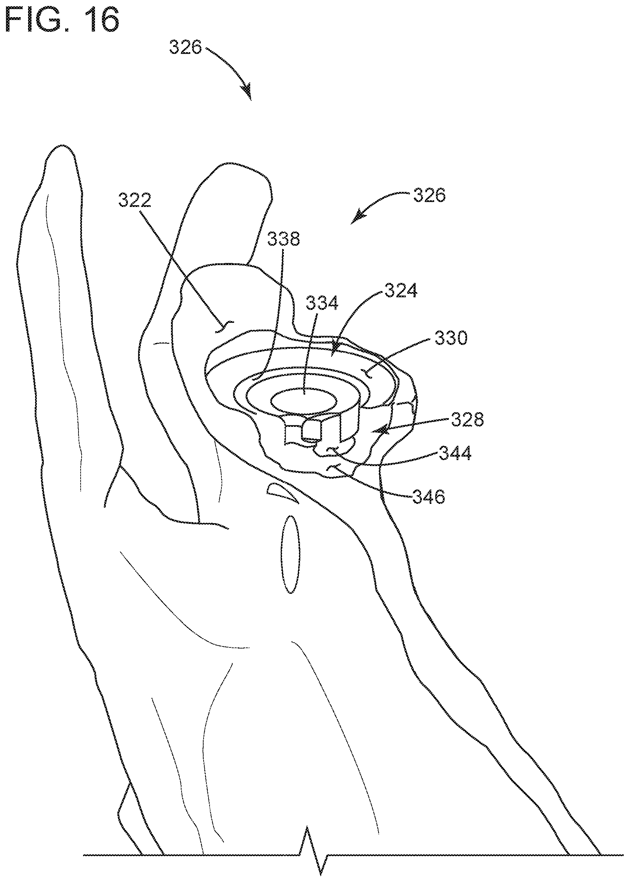

Referring also to FIGS. 15 and 16, a top perspective view (FIG. 15) and side perspective view (FIG. 16) of the defective glenoid cavity 324 is depicted, in accordance with an aspect of the present invention. In FIGS. 15 and 16, the outer surface 322 of the glenoid cavity 324 has been prepared to accept an orthopedic implant system 100 with bone reamer 320.

After surgically exposing the outer surface 322 of the glenoid cavity 324 of a patient (ref. 300 in FIG. 13), the bone reamer 320 may be used to remove a portion of a circular surface 330 into the outer surface 322 of the glenoid cavity 324 (ref. 302 in FIG. 13). The reaming of the portion of the circular surface 330 may be done with the blade member 332 of the bone reamer 320. The portion of the circular surface 330 may, or may not, be a complete circular surface due to bone loss caused by the bone defect 328.

Additionally, the bone reamer 320 may be used to drill a center bore hole 334 into the glenoid cavity 324 that is concentric with the portion of the circular surface 330 (ref. 304 in FIG. 13). The drilling of the center bore hole 334 may be done with the center drill bit 336 of the bone reamer 320.

The bone reamer 320 may cut a portion of a circular groove 338 into the glenoid cavity bone 324 that is concentric with the center bore hole 334 (ref. 306 in FIG. 13). The cutting of the portion of the circular groove 338 may be done with the plurality of cutting pegs 340 of the bone reamer 320. The portion of the circular groove 338 may, or may not, be a complete circular groove for a full 360 degrees due to pre-existing bone loss.

The bone reamer 320 is capable of reaming the portion of the circular surface 330, drilling the center bore hole 334 and cutting the portion of the circular groove 338 substantially simultaneously. However, the portion of the circular surface 330, the center bore hole 334 and the portion of the circular groove 338 may be machined into the glenoid cavity 324 in any sequence using any number of instruments.

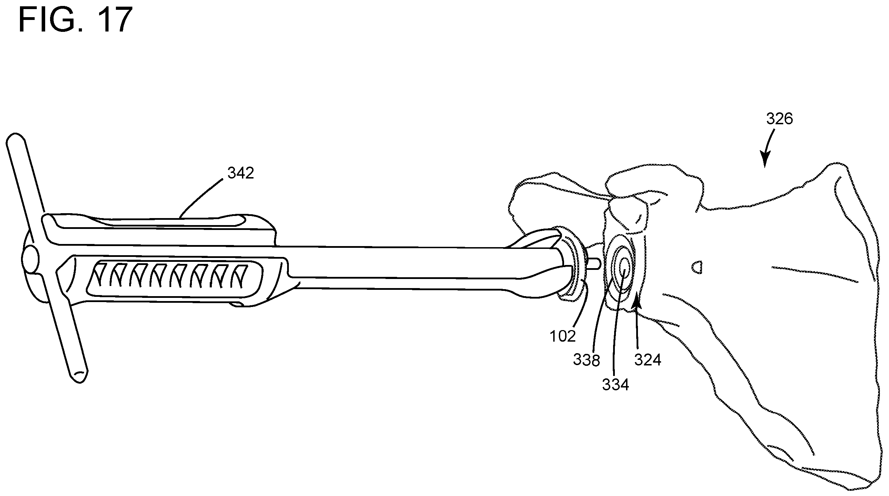

FIG. 17 shows a side perspective view of the base plate 102 of orthopedic implant system 100 being inserted into the prepared glenoid cavity 324 with an insertion tool 342 is depicted. The insertion tool 342 may be used to orient a thru-hole of the base plate 102 of the implant system 100 over the defect 328 in the glenoid cavity bone 324 (ref. 308 in FIG. 13).

Thereafter, the insertion tool 342 may be used to insert the base plate 102 into the center bore hole 334 and the portion of the circular groove 338, such that the thru-hole of the base plate 102 is positioned directly over the defect 324 (ref. 310 in FIG. 13). More specifically, the central bore 114 of the base plate 102 may be positioned, by the insertion tool, directly over the center bore hole 334 within the glenoid cavity 324 and the central screw 104 may be used to anchor the base plate 102 to the glenoid cavity 324 of scapula 326. The arcuate protrusions 122 of the base plate 102 may be press-fit into the circular groove 338 to further anchor the base plate 102 to the glenoid cavity 324. One or more peripheral screws 154 (see FIGS. 19 and 20) may then be used to further anchor the base plate 102 to the glenoid cavity 324.

Thereafter, a flat surface 344 may be reamed into a portion of the surface 346 of the glenoid cavity bone 324 within the defect 328 directly under the thru-hole (ref. 312 in FIG. 13). The reaming may be done by any one of several well-known reaming tools.

Alternatively, no reaming of a surface 344 may be required. This may particularly be the case wherein the augmentation device 200 has an arcuate bottom footing portion 222, a conical bottom footing portion 226 or a threaded bottom footing portion 230.

FIG. 18 shows the orthopedic implant system 100 being implanted into the glenoid cavity 324, in accordance with an aspect of the present invention. Thereafter, the augmentation device 200 extends through the thru-hole (ref. 314 in FIG. 13).

The augmentation device 200 may be fastened to the base plate 102 such that the footing portion 204 of the augmentation device 200 abuts against a surface 346 of the defect 328, and does not penetrate the surface 346 of the glenoid cavity bone 324 (ref. 316 in FIG. 13). More specifically in this case, the augmentation device 200 may be fastened to the base plate 102 by coupling the fastener portion 202 to the peripheral thru-hole 118 such that the foot portion 204 of the augmentation device 200 abuts against the flat surface 344 (see FIGS. 15 and 16) reamed into the surface 346 of the glenoid cavity bone 324 within the defect 328.

When implanted, the augmentation device 200 provides support to the base plate 102 and allows for attachment to the glenoid cavity 324. The augmentation device 200 requires minimal removal of the surface bone within the defect 328. Additionally, the augmentation device 200 allows the same base plate 102 to be used regardless of the deficiencies in the bone. Once the base plate 102 is implanted, then one or more augmentation devices 200 may be inserted depending on the type and severity of the defects encountered.

Referring to FIGS. 19 and 20, a top perspective view (FIG. 19) and a bottom perspective view (FIG. 20) of the orthopedic implant system 100 is depicted in accordance with an aspect of the present invention. Thereafter, a glenosphere 150 may be disposed over the base plate 102 of the implant system 100 for use in a reverse shoulder arthroplasty (ref. 318 in FIG. 13).

The glenosphere 150 may be connected to the base plate 102 by a threaded post 152, which may extend through the coupling member 106 (see FIG. 7) and thread into the female threaded portion 136 of the recess 132 of the central screw 104 (see FIG. 6). The central screw 104 and peripheral screws 154 may be used to anchor the implant system 100 into the scapula 326. The peripheral screws 154 may include first threads 156 configured to engage with threads of the peripheral holes 118 and second threads 158 configured to screw into the scapula 326. The augmentation device 200 may provide support for the base plate 102 when implanted into the scapula 326.

Referring to FIG. 21, a top perspective view of the orthopedic implant system 100 is depicted in accordance with an aspect of the present invention. A glenoid liner 160 may be connected to the base plate 100 rather than the glenoid sphere 150 (ref. 318 in FIG. 13). The glenoid liner 160 may be for use in an anatomic shoulder arthroplasty.

The glenoid liner 160 may be connected to the base plate 102 using several different attaching techniques (not shown). For example, the glenoid liner 160 may have protrusion that match the arcuate slots 120 of the base plate 102, such that the protrusions may be press fit into the slots 120. Alternatively, the glenoid liner 160 may have a flanged end that extends radially inward. The flanged end may cup under the lower surface 112 of the base plate 102 to secure the liner 160 to the base plate 102. To accommodate the glenoid liner 160, the coupling member 106 may be reduced in height so that its top surface is flush, or nearly flush, with the top surface 110 of the base plate, once the coupling member 106 is attached to the base plate 102.

Referring to FIGS. 22 and 23, an example is depicted of a side perspective view (FIG. 22) and a side exploded view (FIG 23) of another example of an orthopedic implant system 400 in accordance with the present invention. In contrast to the implant system 100, the base plate 402, central screw 404 and coupling member 406 are integrally connected as a single monobloc construct. In this implant system 400, there is no central bore 114 (see FIG. 4) that is positioned in the central region 115 of the base plate 102 and to which both the central screw 104 and coupling member 106 may be removably connected. Rather, in implant system 400, the central screw 404 extends from a lower surface 410 of the central region 415 of the base plate 402 and the coupling member 406 extends from a top surface 408 of the central region 415 of the base plate 402. The base plate 402, central screw 404 and coupling member 406 may be cast and/or machined from a single block of material. All other aspects of the implant system 100 and the implant system 400 (including the augmentation devices, such as augmentation device 200) are substantially similar.

As may be recognized by those of ordinary skill in the art based on the teachings herein, numerous changes and modifications may be made to the above-described and other embodiments of the present disclosure without departing from the scope of the invention, which is defined by the claims.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

The invention has been described with reference to the preferred embodiments. It will be understood that the operational embodiments described herein are exemplary of a plurality of possible arrangements to provide the same general features, characteristics, and general system operation. Modifications and alterations will occur to others upon a reading and understanding of the preceding detailed description. It is intended that the invention be construed as including all such modifications and alterations as fall within the scope of the claims.

## Claims

1. An implant system (100) comprising:
a base plate (102) having at least one thru-hole (118, 120);
a central screw (104) operable to extend from a lower surface (112) of the base plate (102) and threadingly anchor the base plate (102) to a bone (10); and
at least one augmentation device (200) configured to extend through the at least one thru-hole (118), the augmentation device (200) comprising:
a post (206) having a first end (208), a second end (210) and a length (212) sized to enable the augmentation device (200) to extend through the at least one thru-hole into a defect (14) in the bone (10) when the base plate (102) is anchored to the bone (10),
a fastener portion (202) positioned on the first end (208) of the post (206), the fastener portion (202) couples the at least one augmentation device (200) to the base plate (102), and
a footing portion (204, 222) positioned on the second end (210) of the post (206), **characterised in that** the footing portion (204, 222) is configured to press against and not penetrate a bone surface within the defect (14) when the fastener portion (202) is coupled to the base plate (102).

2. The implant system (100) of claim 1, comprising:
a central bore (114) disposed in the base plate (102);
wherein the central screw (104) extends through and couples to the central bore (114).

3. The implant system (100) of claim 1, wherein the base plate (102) and central screw (104) are integrally connected as a single monobloc construct.

4. The implant system (100) of claim 1, wherein the footing portion (204, 222) of the augmentation device (200) comprises any one of a flat bottom and an arcuate bottom, wherein the footing portion (204) is operable to press against the surface of the bone (10) within the defect (14).

5. The implant system (100) of claim 1, comprising:
the at least one thru-hole (118, 120) comprising a plurality of threaded peripheral holes (118) disposed circumferentially around the central bore (114); and
the fastener portion (202) comprising a threaded fastener portion (202) having threads (214) configured to engage with the threads of a first peripheral hole (118) of the plurality of peripheral holes (118).

6. The implant system (100) of claim 5, comprising:
at least one peripheral screw (154) operable to extend through any one of the plurality of peripheral holes (118), the at least one peripheral screw (154) comprising:
first threads (156) configured to engage with threads of a second peripheral hole (118) of the plurality of peripheral holes (118), and
second threads (158) configured to screw into the bone (10).

7. The implant system (100) of claim **1,** comprising:
the at least one thru-hole (118, 120) comprising a plurality of arcuate slots (120) disposed circumferentially around the central bore (114); and
the fastener portion (202) configured to engage with a first arcuate slot (120) of the plurality of arcuate slots (120).

8. The implant system (100) of claim 7, comprising:
the first arcuate slot (120) having portions of female threads disposed on opposing side walls of the first arcuate slot (120); and
the fastener portion (202) comprising male threads configured to engage with the portions of female threads of the first arcuate slot (120).

9. The implant system (100) of claim **1,** comprising one of a glenosphere (150) and a glenoid liner (160) operable to be attached to the base plate (102) after the base plate (102) is anchored to the bone (10).

10. The implant system (100) of claim 1, wherein the base plate (102) is circular.

11. The implant system (100) of claim 1, further comprising a coupling member (106), wherein the coupling member (106) threadingly engages the base plate (102) to secure the central screw (104) to the base plate (102).

12. The implant system (100) of claim 1, further comprising a plurality of arcuate protrusions (122), wherein the arcuate protrusions (122) extend from the lower surface (112) of the base plate (102) and are disposed circumferentially around the central bore (114).

13. The implant system (100) of claim 12, wherein the plurality of arcuate protrusions (122) comprise at least one surface having porous material disposed thereon.

14. The implant system (100) of claim 1, wherein the central screw (104) comprises a head portion (128), a proximal portion (124) and a distal portion (126), wherein the head portion (128) is flared, the proximal portion (124) is non-threaded and the distal portion (126) is threaded to engage the bone (10).

## Patentansprüche

1. Ein Implantatsystem (100), das Folgendes beinhaltet:
eine Basisplatte (102) mit mindestens einem Durchgangsloch (118, 120);
eine zentrale Schraube (104), die betrieben werden kann, um sich von einer unteren Oberfläche (112) der Basisplatte (102) zu erstrecken und die Basisplatte (102) an einem Knochen (10) durch Gewinde zu verankern; und
mindestens eine Augmentationsvorrichtung (200), die konfiguriert ist, um sich durch das mindestens eine Durchgangsloch (118) zu erstrecken, wobei die Augmentationsvorrichtung (200) Folgendes beinhaltet:
einen Pfosten (206) mit einem ersten Ende (208), einem zweiten Ende (210) und einer Länge (212), die bemessen ist, um es der Augmentationsvorrichtung (200) zu ermöglichen, sich durch das mindestens eine Durchgangsloch in einen Defekt (14) in dem Knochen (10) zu erstrecken, wenn die Basisplatte (102) an dem Knochen (10) verankert ist,
einen Befestigungsabschnitt (202), der an dem ersten Ende (208) des Pfostens (206) positioniert ist, wobei der Befestigungsabschnitt (202) die mindestens eine Augmentationsvorrichtung (200) mit der Basisplatte (102) koppelt, und
einen Fußabschnitt (204, 222), der an dem zweiten Ende (210) des Pfostens (206) positioniert ist,
**dadurch gekennzeichnet, dass** der Fußabschnitt (204, 222) konfiguriert ist, um gegen eine Knochenoberfläche innerhalb des Defekts (14) zu drücken und diese nicht zu durchdringen, wenn der Befestigungsabschnitt (202) mit der Basisplatte (102) gekoppelt ist.

2. Implantatsystem (100) gemäß Anspruch 1, das Folgendes beinhaltet:
eine zentrale Bohrung (114), die in der Basisplatte (102) angeordnet ist;
wobei sich die zentrale Schraube (104) durch die zentrale Bohrung (114) erstreckt und mit dieser koppelt.

3. Implantatsystem (100) gemäß Anspruch 1, wobei die Basisplatte (102) und die zentrale Schraube (104) integral als eine einzige einstückige Konstruktion verbunden sind.

4. Implantatsystem (100) gemäß Anspruch 1, wobei der Fußabschnitt (204, 222) der Augmentationsvorrichtung (200) eines von einem flachen Boden und einem bogenförmigen Boden beinhaltet, wobei der Fußabschnitt (204) betrieben werden kann, um gegen die Oberfläche des Knochens (10) innerhalb des Defekts (14) zu drücken.

5. Implantatsystem (100) gemäß Anspruch 1, das Folgendes beinhaltet:
das mindestens eine Durchgangsloch (118, 120), das eine Vielzahl von peripheren Gewindelöchern (118), die in Umfangsrichtung um die zentrale Bohrung (114) angeordnet sind, beinhaltet; und
den Befestigungsabschnitt (202), der einen mit Gewinde versehenen Befestigungsabschnitt (202) beinhaltet, der Gewinde (214) aufweist, die konfiguriert sind, um mit den Gewinden eines ersten peripheren Lochs (118) der Vielzahl von peripheren Löchern (118) in Eingriff zu kommen.

6. Implantatsystem (100) gemäß Anspruch 5, das Folgendes beinhaltet:
mindestens eine periphere Schraube (154), die betrieben werden kann, um sich durch eines der Vielzahl von peripheren Löchern (118) zu erstrecken, wobei die mindestens eine periphere Schraube (154) Folgendes beinhaltet:
erste Gewinde (156), die konfiguriert sind, um mit Gewinden eines zweiten peripheren Lochs (118) der Vielzahl von peripheren Löchern (118) in Eingriff zu kommen, und
zweite Gewinde (158), die konfiguriert sind, um in den Knochen (10) geschraubt zu werden.

7. Implantatsystem (100) gemäß Anspruch 1, das Folgendes beinhaltet:
das mindestens eine Durchgangsloch (118, 120), das eine Vielzahl von bogenförmigen Schlitzen (120), die in Umfangsrichtung um die zentrale Bohrung (114) angeordnet sind, beinhaltet; und
den Befestigungsabschnitt (202), der konfiguriert ist, um mit einem ersten bogenförmigen Schlitz (120) der Vielzahl von bogenförmigen Schlitzen (120) in Eingriff zu kommen.

8. Implantatsystem (100) gemäß Anspruch 7, das Folgendes beinhaltet:
den ersten bogenförmigen Schlitz (120), der Abschnitte von Innengewinden aufweist, die an gegenüberliegenden Seitenwänden des ersten bogenförmigen Schlitzes (120) angeordnet sind; und
den Befestigungsabschnitt (202), der Außengewinde beinhaltet, die konfiguriert sind, um mit den Abschnitten von Innengewinden des ersten bogenförmigen Schlitzes (120) in Eingriff zu kommen.

9. Implantatsystem (100) gemäß Anspruch 1, das eines von einer Glenosphäre (150) und einer Glenoidauskleidung (160) beinhaltet, die betrieben werden kann, um an der Basisplatte (102) angebracht zu werden, nachdem die Basisplatte (102) an dem Knochen (10) verankert ist.

10. Implantatsystem (100) gemäß Anspruch 1, wobei die Basisplatte (102) kreisförmig ist.

11. Implantatsystem (100) gemäß Anspruch 1, das ferner ein Kopplungselement (106) beinhaltet, wobei das Kopplungselement (106) die Basisplatte (102) durch Gewinde in Eingriff nimmt, um die zentrale Schraube (104) an der Basisplatte (102) zu sichern.

12. Implantatsystem (100) gemäß Anspruch 1, das ferner eine Vielzahl von bogenförmigen Vorsprüngen (122) beinhaltet, wobei sich die bogenförmigen Vorsprünge (122) von der unteren Oberfläche (112) der Basisplatte (102) erstrecken und in Umfangsrichtung um die zentrale Bohrung (114) angeordnet sind.

13. Implantatsystem (100) gemäß Anspruch 12, wobei die Vielzahl von bogenförmigen Vorsprüngen (122) mindestens eine Oberfläche beinhaltet, auf der poröses Material angeordnet ist.

14. Implantatsystem (100) gemäß Anspruch 1, wobei die zentrale Schraube (104) einen Kopfabschnitt (128), einen proximalen Abschnitt (124) und einen distalen Abschnitt (126) beinhaltet, wobei der Kopfabschnitt (128) aufgeweitet ist, der proximale Abschnitt (124) kein Gewinde aufweist und der distale Abschnitt (126) ein Gewinde aufweist, um mit dem Knochen (10) in Eingriff zu kommen.

## Revendications

1. Un système d'implant (100) comprenant :
une plaque de base (102) ayant au moins un trou débouchant (118, 120) ;
une vis centrale (104) utilisable pour s'étendre à partir d'une surface inférieure (112) de la plaque de base (102) et ancrer par filetage la plaque de base (102) à un os (10) ; et au moins un dispositif d'augmentation (200) configuré pour s'étendre à travers l'au moins un trou débouchant (118), le dispositif d'augmentation (200) comprenant :
un tenon (206) ayant une première extrémité (208), une deuxième extrémité (210) et une longueur (212) dimensionnée pour permettre au dispositif d'augmentation (200) de s'étendre à travers l'au moins un trou débouchant jusque dans un défaut (14) dans l'os (10) lorsque la plaque de base (102) est ancrée à l'os (10),
une portion de fixation (202) positionnée sur la première extrémité (208) du tenon (206), la portion de fixation (202) accouplant l'au moins un dispositif d'augmentation (200) à la plaque de base (102), et
une portion d'appui (204, 222) positionnée sur la deuxième extrémité (210) du tenon (206),
**caractérisé en ce que** la portion d'appui (204, 222) est configurée pour s'appuyer contre et ne pas pénétrer une surface d'os au sein du défaut (14) lorsque la portion de fixation (202) est accouplée à la plaque de base (102).

2. Le système d'implant (100) de la revendication 1, comprenant :
un alésage central (114) disposé dans la plaque de base (102) ;
dans lequel la vis centrale (104) s'étend à travers et s'accouple à l'alésage central (114).

3. Le système d'implant (100) de la revendication 1, dans lequel la plaque de base (102) et la vis centrale (104) sont reliées solidairement sous la forme d'une construction monobloc unique.

4. Le système d'implant (100) de la revendication 1, dans lequel la portion d'appui (204, 222) du dispositif d'augmentation (200) comprend l'un quelconque d'un fond plat et d'un fond arqué, la portion d'appui (204) étant utilisable pour s'appuyer contre la surface de l'os (10) au sein du défaut (14).

5. Le système d'implant (100) de la revendication 1, comprenant :
l'au moins un trou débouchant (118, 120) comprenant une pluralité de trous périphériques (118) filetés disposés circonférentiellement autour de l'alésage central (114) ; et
la portion de fixation (202) comprenant une portion de fixation (202) filetée ayant des filets (214) configurés pour se mettre en prise avec les filets d'un premier trou périphérique (118) de la pluralité de trous périphériques (118).

6. Le système d'implant (100) de la revendication 5, comprenant :
au moins une vis périphérique (154) utilisable pour s'étendre à travers l'un quelconque de la pluralité de trous périphériques (118), l'au moins une vis périphérique (154) comprenant :
des premiers filets (156) configurés pour se mettre en prise avec des filets d'un deuxième trou périphérique (118) de la pluralité de trous périphériques (118), et
des deuxièmes filets (158) configurés pour se visser dans l'os (10).

7. Le système d'implant (100) de la revendication 1, comprenant :
l'au moins un trou débouchant (118, 120) comprenant une pluralité d'encoches arquées (120) disposées circonférentiellement autour de l'alésage central (114) ; et
la portion de fixation (202) configurée pour se mettre en prise avec une première encoche arquée (120) de la pluralité d'encoches arquées (120).

8. Le système d'implant (100) de la revendication 7, comprenant :
la première encoche arquée (120) ayant des portions de filets femelles disposées sur des parois latérales opposées de la première encoche arquée (120) ; et
la portion de fixation (202) comprenant des filets mâles configurés pour se mettre en prise avec les portions de filets femelles de la première encoche arquée (120).

9. Le système d'implant (100) de la revendication 1, comprenant soit une glénosphère (150), soit un bourrelet glénoïdien (160) utilisable pour être attaché(e) à la plaque de base (102) après que la plaque de base (102) a été ancrée à l'os (10).

10. Le système d'implant (100) de la revendication 1, dans lequel la plaque de base (102) est circulaire.

11. Le système d'implant (100) de la revendication 1, comprenant en outre un élément d'accouplement (106), l'élément d'accouplement (106) se mettant en prise par filetage avec la plaque de base (102) pour assujettir la vis centrale (104) à la plaque de base (102).

12. Le système d'implant (100) de la revendication 1, comprenant en outre une pluralité de saillies arquées (122), les saillies arquées (122) s'étendant à partir de la surface inférieure (112) de la plaque de base (102) et étant disposées circonférentiellement autour de l'alésage central (114).

13. Le système d'implant (100) de la revendication 12, dans lequel la pluralité de saillies arquées (122) comprennent au moins une surface ayant un matériau poreux disposé sur celle-ci.

14. Le système d'implant (100) de la revendication 1, dans lequel la vis centrale (104) comprend une portion formant tête (128), une portion proximale (124) et une portion distale (126), la portion formant tête (128) étant évasée, la portion proximale (124) étant non filetée et la portion distale (126) étant filetée pour se mettre en prise avec l'os (10).
